# EUROPÄISCHE PATENTSCHRIFT

(11) **EP 3 568 473 B1**
(45) Veröffentlichungstag und Bekanntmachung des Hinweises auf die Patenterteilung: **14.12.2022**
(21) Anmeldenummer: 17804096.0
(22) Anmeldetag: 08.11.2017
(51) Int. Cl.: C12N 15/10

(54) **VERFAHREN ZUM EXTRAHIEREN VON DNS**
METHOD FOR EXTRACTING DEOXYRIBONUCLEIC ACID
PROCÉDÉ D'EXTRACTION D'ADN

(30) Priorität: 11.01.2017 DE 102017200332
(43) Veröffentlichungstag der Anmeldung: 20.11.2019
(73) Patentinhaber: Füll Process SA, 8595 Altnau (CH)
(72) Erfinder: BRINZ, Thomas, 73266 Bissingen A.D. Teck (DE); FRANK, Joel Johannes, 77656 Offenburg (DE)
(74) Vertreter: DREISS Patentanwälte PartG mbB
(86) Internationale Anmeldenummer: PCT/EP2017/078538
(87) Internationale Veröffentlichungsnummer: WO 2018/130319

(56) Entgegenhaltungen:
- WO-A1-2015/018647
- DE-T2- 69 524 560
- US-B2- 8 703 931

## Beschreibung

### Stand der Technik

Die vorliegende Erfindung betrifft ein Verfahren zum Extrahieren von DNS wie in den Ansprüchen definiert.

Insbesondere wird ein Verfahren zum automatisierten Extrahieren beschrieben, welches besonders vorteilhaft zum automatisierten Aufreinigen von DNS ist. Im Rahmen dieser Erfindung wird unter DNS Desoxyribonukleinsäure verstanden.

Aus dem Stand der Technik sind Verfahren zum Extrahieren von DNS bekannt. Dazu erfolgt ein Austrennen von DNS aus Zellmaterial. Anschließend werden sogenannte Magnetik Beads verwendet, bei denen es sich um kleine magnetische Partikel handelt, welche mit der DNS eine Bindung eingehen. Auf diese Weise ist ein Reinigen von DNS ermöglicht. Die Methode beruht darauf, dass DNS in hochkonzentrierten Salzlösungen reversibel an solche Partikel, beispielsweise an Siliziumdioxid, bindet.

Zunächst werden die Zellen aufgebrochen und es wird ein Lysat gebildet, welches sowohl die freigesetzte DNS als auch restliche Substanzen der Zellen beinhaltet. In das Gemisch werden chaotrope Salze gegeben, die die regelmäßige Struktur des Wassers beeinträchtigen oder ganz auflösen und hydrophobe Interaktionen unterbinden.

In die Mischung werden die beschriebenen Beads gegeben, welche mit Siliziumdioxid oder einer anderen stark DNS-adsorbierenden Substanz beschichtet sind. Die DNS geht daher eine spezifische Bindung mit dem Siliziumdioxid an der Oberfläche der Beads ein. Die magnetische Eigenschaft der Beads ermöglicht eine magnetische Fixierung der DNS. Gleichzeitig können restliche Substanzen, wie insbesondere Zellabfälle, aus dem Gemisch herausgewaschen werden. Um die Bindung der DNS an die Beads aufzulösen, wird im letzten Schritt mit einer Lösung gespült, welche keine chaotrope Salze mehr enthält. Durch einen Magneten können nun die Beats von der DNS getrennt werden, sodass die DNS für weitere Schritte bereitsteht. US8703931 beschreibt ein Verfahren zum Extrahieren von DNS, umfassend die Schritte: Bereitstellen eines Lysats in einem Probengefäß, Zufügen einer DNS adsorbierenden Substanz zu dem Probengefäß, Zuführen eines Waschfluids in das Probengefäß und Abführen des Waschfluids aus dem Probengefäß wobei die DNS adsorbierende Substanz durch einen Magneten rückhaltbar ist.

Ein Verfahren zum kontinuierlichen Extrahieren von DNS ist beispielsweise auch in der DE 10 2013 215 575 A1 beschrieben.

### Offenbarung der Erfindung

Das erfindungsgemäße Verfahren ermöglicht ein vollautomatisiertes Extrahieren und/oder Aufreinigen von DNS ohne Eingreifen eines Menschen. So werden insbesondere zu Beginn Zellmaterialien in speziellen Probengefäßen zur Verfügung gestellt. Eine vollautomatisiert arbeitende Maschine kann anschließend die Extraktion und/oder Aufreinigung der DNS nach vorgegebenen Protokollen und/oder Abläufen umsetzen. Hierfür muss lediglich jeder Probe ein Ablauf zugeordnet werden, was der einzige Eingriff durch einen Menschen darstellt. Insbesondere ist das Verfahren dadurch vereinfacht, dass die DNS das Probengefäß während des gesamten Vorgangs nicht verlässt.

Das Verfahren zum Extrahieren von DNS umfasst die folgenden Schritte:
Zunächst erfolgt ein Bereitstellen eines Lysats in einem Probengefäß. Bei einem Lysat handelt es sich insbesondere um eine Emulsion, in der beschädigtes Zellmaterial vorhanden ist. Insbesondere ist eine äußere Zellmembran der Zellen des Zellmaterials beschädigt oder aufgelöst. Anschließend erfolgt das Zufügen einer DNS-adsorbierenden Substanz zu dem Probengefäß. DNS-adsorbierende Substanzen sind bekannt. Vorteilhafterweise handelt es sich bei der DNS-adsorbierenden Substanz um eine solche Substanz, die von einem Waschfilterelement rückhaltbar ist. Das Waschfilterelement wird in einem nächsten Schritt auf das Probengefäß aufgebracht. Anschließend erfolgt ein Zuführen eines Waschfluids in das Probengefäß durch das Waschfilterelement und danach ein Abführen des Waschfluids aus dem Probengefäß durch das Waschfilterelement. Auf diese Weise ist erreicht, dass das Waschfilterelement DNS aufgrund der DNS-adsorbierenden Substanz zurückhält, gleichzeitig jedoch durchlässig ist für Substanzen, die ausgewaschen werden sollen. Bei solchen Substanzen handelt es sich insbesondere um Zellschrott, das heißt, Reste des Zellmaterials, sowie sonstige Komponenten, die nicht von der DNS-adsorbierenden Substanz adsorbiert werden. Die DNS verbleibt in dem Probengefäß, da diese aufgrund der DNS-adsorbierenden Substanz von dem Waschfilterelement zurückgehalten wird. Das Zuführen und Abführen des Waschfluids ist durch das Verfahren vereinfacht, insbesondere optimal automatisierbar. Insbesondere erfolgt das Vereinfachen dadurch, dass die DNS das Probengefäß während des gesamten Vorgangs nicht verlässt. Vielmehr wird stets Waschfluid zugeführt und abgeführt. Das Zuführen und Abführen erfolgt durch das Waschfilterelement, sodass lediglich eine einzige Verbindung zwischen äußerer Umgebung und Probengefäß vorhanden sein muss. Besagte Verbindung wird durch das Waschfilterelement realisiert.

Das erfindungsgemäße Verfahren zum Extrahieren von DNS umfasst vorteilhafterweise das Auslösen der DNS aus einem Zellmaterial und einen Waschvorgang zum Aufreinigen der DNS. Das Auslösen aus dem Zellmaterial erfolgt mittels des zuvor beschriebenen Schritts des Bereitstellens des Lysats. Der Waschvorgang erfolgt durch die übrigen zuvor beschriebenen Schritte. Auf diese Weise kann DNS zuverlässig extrahiert werden, wobei der gesamte dazu erforderliche Vorgang automatisierbar ist und somit rasch und kostengünstig abläuft. Bei dem erfindungsgemäßen Verfahren werden bevorzugt sämtliche Prozessschritte seriell durchgeführt.

Die Unteransprüche haben bevorzugte Weiterbildungen der Erfindung zum Inhalt.

Bevorzugt ist vorgesehen, dass der Schritt des Bereitstellens des Lysats die folgenden Schritte umfasst: Zunächst erfolgt ein Einbringen von Zellmaterial in ein Zusatzprobengefäß. Außerdem wird ein Mahlelement in das Zusatzprobengefäß eingebracht. Bei dem Mahlelement handelt es sich insbesondere um eine Mahlkugel. Anschließend erfolgt ein Zerstören des Zellmaterials durch Bewegung des Zusatzprobengefäßes. Dazu wird das Zusatzprobengefäß vorteilhafterweise verschlossen. Das Bewegen erfolgt insbesondere durch hochfrequentes alternierendes Bewegen des Zusatzprobengefäßes. In einer besonders vorteilhaften Ausgestaltung ist das Zusatzprobengefäß zylinderförmig ausgebildet, wobei ein Bewegen insbesondere entlang einer Mittelachse der Zylinderform erfolgt. Auf diese Weise wird die Mahlkugel zum Zerstören der Zellen des Zellmaterials verwendet. Schließlich erfolgt ein Schritt des Ausfilterns des Lysats aus dem Zusatzprobengefäß in das zuvor beschriebene Probengefäß durch ein Lysatfilterelement. Somit findet während des gesamten Verfahrens bevorzugt ein einziger Wechsel zwischen dem Zusatzprobengefäß und dem Probengefäß statt, wobei das ausgefilterte Lysat das Probengefäß während des gesamten Verfahrens nicht mehr verlässt. Insbesondere wird somit sichergestellt, dass nicht zerstörtes Zellmaterial sowie grober Zellschrott, der erheblich größer als die DNS ist, in dem Zusatzprobengefäß verbleibt. Das in dem Probengefäß bereitgestellte Lysat muss daher lediglich mittels des zuvor beschriebenen Aufreinigens durch Zuführen und Abführen eines Waschfluids gereinigt werden. Anschließend steht die DNS für weitere Schritte zur Verfügung. Auch die zuvor beschriebenen Schritte des Einbringens von Zellmaterial in das Zusatzprobengefäß, das Einbringen des Mahlelements, das Zerstören des Zellmaterials und das Ausfiltern des Lysats sind einfach und aufwandsarm durchführbar. Somit sind besagte Schritte einfach und aufwandsarm automatisierbar, um rasch und zuverlässig ein Bereitstellen von Lysat in dem Probengefäß zu ermöglichen. Wiederum ist bevorzugt vorgesehen, dass keinerlei Eingriffe eines Menschen erforderlich sind.

Vorteilhafterweise ist vorgesehen, dass das Waschfilterelement oder das Lysatfilterelement in einem Stempel angeordnet ist. Dabei ist vorgesehen, dass der Stempel in das Probengefäß oder das Zusatzprobengefäß einsteckbar ist. Weiterhin ist vorgesehen, dass der Stempel ein Zuführen und/oder Abführen von Waschfluid erlaubt. Dies wird dadurch erreicht, dass durch den Stempel das Waschfluid in das Probengefäß gesaugt und aus dem Probengefäß gepresst wird. Der Stempel weist somit eine Öffnung auf, die ein Ansaugen und Auspressen von Waschfluid ermöglicht. In der Öffnung befindet sich außerdem das Waschfilterelement, sodass das zuvor beschriebene Zuführen und Abführen von Waschfluid nur durch das Waschfilterelement möglich ist. Alternativ ist der Stempel derart ausgebildet, dass durch den Stempel das Lysat von dem Zusatzprobengefäß in das Probengefäß gepresst wird. Wiederum weist der Stempel bevorzugt eine Öffnung auf, durch die das Lysat pressbar ist. Innerhalb der Öffnung befindet sich das Lysatfilterelement, wodurch sichergestellt ist, dass nur das ausgefilterte Lysat in das Probengefäß übertragen wird. Das Ausbilden des Stempels vereinfacht somit die genannten Schritte. Der Stempel ist insbesondere derart ausgebildet, dass dieser eine Zylinderform aufweist. Das Probengefäß und das Zusatzprobengefäß weisen vorteilhafterweise ebenfalls eine Zylinderform auf, wobei der Stempel in das Probengefäß oder das Zusatzprobengefäß einführbar ist. Besonders vorteilhaft entsteht an einer äußeren Umfangsfläche des Stempels und an einer inneren Umfangsfläche des Probengefäßes oder des Zusatzprobengefäßes eine fluiddichte Abdichtung, sodass ein Fluidaustausch zwischen Probengefäß und Umgebung ausschließlich durch die Öffnung des Stempels erfolgen kann.

Der Stempel und das Probengefäß oder das Zusatzprobengefäß bilden besonders vorteilhaft eine Spritze. Somit ist durch relatives Bewegen von Stempel und Probengefäß oder Zusatzprobengefäß zueinander ein Ansaugen von externen Fluiden in das Probengefäß ermöglicht, gleichzeitig kann durch eine solche relative Bewegung der Inhalt des Probengefäßes ausgepresst werden. Somit ist einfach und aufwandsarm Fluid in das Probengefäß einbringbar und aus dem Probengefäß entnehmbar. Außerdem ermöglicht das Ausbilden der Spritze ein genaues Dosieren des aufzunehmenden oder abzugebenden Fluids. Schließlich ist das Zuführen und Abführen von Fluid zu oder von dem Probengefäß oder dem Zusatzprobengefäß einfach automatisierbar, sodass ein menschliches Eingreifen nicht notwendig ist. Da das Zuführen und Abführen von Waschfluid ausschließlich durch das Waschfilterelement erfolgt, ist die Spritze, insbesondere der Stempel, sehr einfach aufgebaut, da lediglich eine einzige Öffnung bereitgestellt werden muss, durch die das Zuführen und Abführen des Waschfluids erfolgt.

Während des gesamten Verfahrens, insbesondere während des Waschvorgangs, wird das Probengefäß vorteilhafterweise in einer Haltevorrichtung gehalten. Alternativ oder zusätzlich wird das Zusatzprobengefäß zumindest während des Bereitstellens des Lysats in einem Zusatzhalteelement gehalten. Das Probengefäß ist in dem Halteelement bevorzugt fixierbar. Ebenso ist das Zusatzprobengefäß in dem Zusatzhalteelement bevorzugt fixierbar. Somit kann durch Bewegen des Halteelements und/oder des Zusatzhalteelements eine Bewegung des Probengefäßes oder des Zusatzprobengefäßes erreicht werden. Da durch die Verwendung des zuvor beschriebenen Stempels vorteilhafterweise Fluiddichtigkeit besteht, kann somit auch ein Verdrehen oder Verkippen des Probengefäßes oder des Zusatzprobengefäßes erfolgen. Dabei ist sichergestellt, dass das Probengefäß sicher und zuverlässig in der Haltevorrichtung gehalten ist, während das Zusatzprobengefäß sicher und zuverlässig in der Zusatzvorrichtung gehalten ist.

Besonders vorteilhaft ist eine Vielzahl von Probengefäßen gleichzeitig in dem Halteelement gehalten. Ebenso ist besonders vorteilhaft, dass eine Vielzahl von Zusatzprobengefäßen in dem Zusatzhalteelement gehalten ist. Die zuvor beschriebenen Verfahrensschritte werden somit bevorzugt gleichzeitig auf eine Vielzahl von Probengefäßen oder Zusatzprobengefäßen angewandt. Unter einer Vielzahl ist insbesondere eine gerade Anzahl an Probengefäßen oder Zusatzprobengefäßen zu verstehen. Besonders vorteilhaft ist unter einer Vielzahl eine Anzahl von zumindest vier zu verstehen. All die Probengefäße sind somit von einem einzigen Halteelement gehalten, wobei alle Probengefäße in dem Halteelement fixierbar sind. Ein Fixieren oder ein Lösen der Probengefäße in dem Halteelement erfolgt vorteilhafterweise gleichzeitig. Analog gilt dies auch für die Zusatzprobengefäße, sodass die Vielzahl von Zusatzprobengefäßen in einem einzigen Zusatzhalteelement gehalten ist. Auch hier kann das Fixieren oder Freigeben der Zusatzprobengefäße gleichzeitig erfolgen. Insbesondere ist somit ermöglicht, dass eine große Vielzahl von einzelnen Probengefäßen oder Zusatzprobengefäßen gleichzeitig handhabbar ist. Somit ist das Verfahren vorteilhafterweise automatisierbar, sodass ein hoher Durchsatz in minimierter Zeit erreicht werden kann.

In einer weiteren besonders bevorzugten Ausführungsform ist vorgesehen, dass eine Vielzahl von Stempeln an einem Trägerelement fixiert ist. Die Vielzahl von Stempeln ist vorteilhafterweise identisch zu der Vielzahl von Probengefäßen und/oder Zusatzprobengefäßen. Somit lässt sich die Vielzahl von Stempeln gleichzeitig in die Vielzahl von Probengefäßen und/oder Zusatzprobengefäßen einstecken. Dies verbessert wiederum die Automatisierbarkeit, sodass eine Vielzahl von Probengefäßen und/oder Zusatzprobengefäßen gleichzeitig verarbeitet werden können. Die Stempel sind insbesondere formschlüssig mit dem Trägerelement verbunden.

Besonders vorteilhaft erfolgt das Zuführen von Waschfluid und/oder das Abführen von Waschfluid und/oder das Ausfiltern des Lysats durch Bewegen von Trägerelement und Halteelement oder Zusatzhalteelement relativ zueinander. Besonders vorteilhaft wird das Halteelement relativ zu dem Trägerelement bewegt. Auf diese Weise ist ein vereinfachtes Zuführen oder Abführen von Waschfluid ermöglicht, ebenso wie ein vereinfachtes Ausfiltern des Lysats. Insbesondere ist somit eine vereinfachte Automatisierbarkeit erreicht, wobei gleichzeitig eine große Vielzahl von Probengefäßen und/oder Zusatzprobengefäßen gleichzeitig verarbeitet werden kann.

In einer weiteren vorteilhaften Ausführungsform ist vorgesehen, dass das Zuführen von Waschfluid durch gleichzeitiges Ansaugen von Waschfluid über die Vielzahl von Stempeln aus einem gemeinsamen Reservoir erfolgt. Alternativ erfolgt das Ansaugen über die Vielzahl von Stempeln aus jeweiligen Einzelreservoirs, wobei jedem Stempel ein Einzelreservoir zugeordnet ist. Das Zuführen von Waschfluid erfolgt vorteilhafterweise derart, dass die Öffnung der Stempel in Kontakt mit dem Waschfluid gebracht werden, insbesondere in das Waschfluid eingetaucht werden. Anschließend erfolgt eine Bewegung des Halteelements, wobei sich das Halteelement von dem Trägerelement und dem Reservoir oder der Vielzahl von Einzelreservoirs entfernt. Auf diese Weise wird ein Unterdruck innerhalb der Probengefäße erzeugt, wodurch das Waschfluid durch die Stempel angesaugt wird. Zum Abführen des Waschfluids wird eine gegengerichtete Bewegung des Halteelements durchgeführt, sodass das Waschfluid aus den Probengefäßen gepresst wird. Besonders vorteilhaft erfolgt ein Abführen des Waschfluids in einen gesonderten Abfallbehälter.

Vorteilhafterweise ist vorgesehen, dass das Reservoir durch zumindest ein Unterteilungselement in einzelne Teilreservoirs für jeden Stempel unterteilt wird. Das zumindest eine Unterteilungselement ist vorteilhafterweise herausnehmbar und somit aus dem Reservoir entfernbar. Somit kann das Reservoir mit dem Waschfluid einfach befüllt werden, wobei anschließend durch das Unterteilungselement Teilreservoirs abgegrenzt sind, sodass jedem Stempel ein eigenes Teilreservoir zur Verfügung steht.

Die zuvor beschriebenen Schritte des Zuführens von Waschfluid und des Abführens von Waschfluid werden vorteilhafterweise wiederholt durchgeführt. Die Wiederholung der Schritte erfolgt besonders vorteilhaft mit unterschiedlichem Waschfluid. Somit erfolgt ein sicheres und zuverlässiges Reinigen der DNS. Insbesondere kann durch das wiederholte Zuführen und Abführen von, insbesondere unterschiedlichen, Waschfluiden die DNS vollumfänglich gereinigt werden. Somit erlaubt das Verfahren ein sicheres und zuverlässiges Bereitstellen von reiner DNS. Für das wiederholende Zuführen und Abführen von Waschfluid kann insbesondere stets derselbe Stempel verwendet werden. Der Stempel kann vor einem erneuten Verwenden zum Zuführen und Abführen von Waschfluid gespült und/oder gereinigt werden. Alternativ kann auch für jedes erneute Durchführen der Schritte Zuführen und Abführen von Waschfluid ein neuer Stempel verwendet werden. Da die Schritte des Zuführens und Abführens von Waschfluid durch das zuvor beschriebene Verwenden des Stempels einfach und aufwandsarm automatisierbar sind, kann eine beliebige Anzahl von Wiederholungen durchgeführt werden, ohne die gesamte Prozesszeit für das Extrahieren von DNS merklich zu verlängern. Ebenso ist verhindert, dass ein Aufwand zum Durchführen des Verfahrens, das heißt, ein Aufwand zum Durchführen des Extrahierens der DNS, unwirtschaftlich hoch wird.

Besonders vorteilhaft erfolgt nach dem Zuführen des Waschfluids und vor dem Abführen des Waschfluids ein Auflockern des Filterkuchens, der sich an dem Waschfilterelement befindet. Dazu wird dem Probengefäß Luft zugeführt. Das Zuführen der Luft erfolgt insbesondere auf dieselbe Weise wie das Zuführen des Waschfluids. Somit kann sich der Filterkuchen optimal mit dem Waschfluid durchmischen.

Das Zuführen des Waschfluids und das Abführen des Waschfluids dient insbesondere zum Reinigen der DNS. Daher ist besonders vorteilhaft vorgesehen, dass nach dem Zuführen des Waschfluids und vor dem Abführen des Waschfluids das Probengefäß, insbesondere hochfrequent, kreisförmig oder alternierend bewegt wird. Auf diese Weise wird ein vorhandener Filterkuchen an dem Waschfilterelement gelöst und/oder gelockert. Auf diese Weise wird erreicht, dass der Filterkuchen mit dem Waschfluid optimal durchmischt wird. Da der Filterkuchen aus der DNS gebildet ist, findet somit ein optimales Waschen der DNS statt.

In einer besonders vorteilhaften Ausführungsform ist vorgesehen, dass das Waschfilterelement und das Lysatfilterelement identisch sind. Dies bedeutet, dass lediglich eine einzige Art von Filterelement bereitzustellen ist, um das gesamte Verfahren wie zuvor beschrieben durchzuführen. Dies bedeutet, dass lediglich eine einzige Art von Stempeln bereitzustellen ist. Das Waschfilterelement und das Lysatfilterelement dienen vorteilhafterweise zum Rückhalten von DNS lediglich dann, wenn diese mit einer DNS-adsorbierenden Substanz versehen sind. Somit ist die DNS einfach und aufwandsarm extrahierbar, da stets dasselbe Filterelement für unterschiedliche Verfahrensschritte verwendet werden kann.

Nach dem Abführen des Waschfluids wird bevorzugt eine Zusatzsubstanz in das Probengefäß eingegeben. Das Probengefäß wird dazu insbesondere mit einer solchen Zusatzsubtanz versetzt, die die DNS-adsorbierende Substanz von der DNS löst. Dies bedeutet, dass nach dem Zugeben der Zusatzsubstanz die DNS für weitere Schritte zur Verfügung steht. Die DNS ist dabei durch das zuvor durchgeführte Verfahren hochwertig gereinigt. Zusatzsubstanzen zum Lösen der DNS-adsorbierenden Substanz von der DNS sind aus dem Stand der Technik bekannt.

### Kurze Beschreibung der Zeichnung(en)

Nachfolgend werden Ausführungsbeispiele der Erfindung unter Bezugnahme auf die begleitenden Zeichnungen im Detail beschrieben. In den Zeichnungen ist:
- Figur 1: eine schematische Ansicht eines Ablaufs eines Verfahrens gemäß einem Ausführungsbeispiel der Erfindung,
- Figur 2: eine schematische Ansicht einer ersten Alternative eines Waschvorgangs des Verfahrens gemäß dem Ausführungsbeispiel der Erfindung,
- Figur 3: eine schematische Ansicht der zum Durchführen des Waschvorgangs gemäß der ersten Alternative verwendeten Komponenten,
- Figur 4: eine schematische Ansicht einer zweiten Alternative eines Waschvorgangs des Verfahrens gemäß dem Ausführungsbeispiel der Erfindung,
- Figur 5: eine schematische Ansicht der zum Durchführen des Waschvorgangs gemäß der zweiten Alternative verwendeten Komponenten,
- Figur 6: eine schematische Ansicht einer dritten Alternative eines Waschvorgangs des Verfahrens gemäß dem Ausführungsbeispiel der Erfindung,
- Figur 7: eine schematische Ansicht der zum Durchführen des Waschvorgangs gemäß der dritten Alternative verwendeten Komponenten,
- Figur 8: eine schematische Ansicht einer vierten Alternative eines Waschvorgangs des Verfahrens gemäß dem Ausführungsbeispiel der Erfindung,
- Figur 9: eine schematische Ansicht der zum Durchführen des Waschvorgangs gemäß der vierten Alternative verwendeten Komponenten,
- Figur 10: eine schematische Ansicht einer fünften Alternative eines Waschvorgangs des Verfahrens gemäß dem Ausführungsbeispiel der Erfindung,
- Figur 11: eine erste schematische Ansicht der zum Durchführen des Waschvorgangs gemäß der fünften Alternative verwendeten Komponenten,
- Figur 12: eine zweite schematische Ansicht der zum Durchführen des Waschvorgangs gemäß der fünften Alternative verwendeten Komponenten,
- Figur 13: eine schematische Ansicht einer sechsten Alternative eines Waschvorgangs des Verfahrens gemäß dem Ausführungsbeispiel der Erfindung,
- Figur 14: eine erste schematische Ansicht der zum Durchführen des Waschvorgangs gemäß der sechsten Alternative verwendeten Komponenten,
- Figur 15: eine zweite schematische Ansicht der zum Durchführen des Waschvorgangs gemäß der sechsten Alternative verwendeten Komponenten,
- Figur 16: eine schematische Ansicht eines ersten Mischvorgangs während des Verfahrens gemäß dem Ausführungsbeispiel der Erfindung,
- Figur 17: eine schematische Ansicht eines zweiten Mischvorgangs während des Verfahrens gemäß dem Ausführungsbeispiel der Erfindung, und
- Figur 18: eine schematische Ansicht eines dritten Mischvorgangs während des Verfahrens gemäß dem Ausführungsbeispiel der Erfindung.

### Ausführungsformen der Erfindung

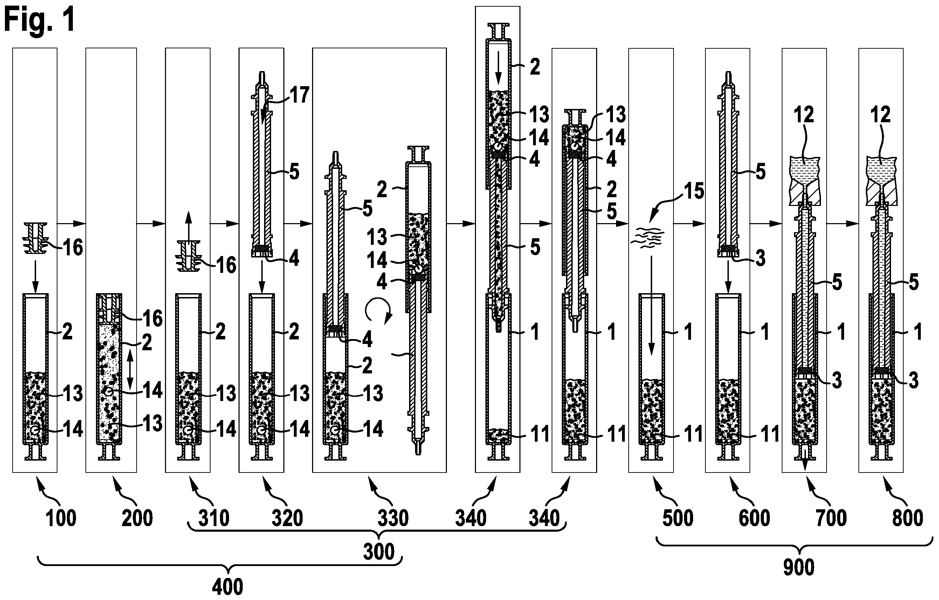

Figur 1 zeigt schematisch einen Ablauf eines Verfahrens gemäß einem Ausführungsbeispiel der Erfindung. Das Verfahren unterteilt sich insbesondere in zwei Schritte, einen Schritt des Bereitstellens 100 von Lysat 11 in einem Probengefäß 1 sowie einen Waschvorgang 900, um die DNS innerhalb des Lysats 11 zu reinigen.

Der Schritt des Bereitstellens 400 des Lysats 11 umfasst insbesondere mehrere Unterschritte. So erfolgt zunächst ein Einbringen 100 von Zellmaterial 13 sowie von einem Mahlelement 14 in ein Zusatzprobengefäß 2. Bei dem Mahlelement 14 handelt es sich insbesondere um eine Mahlkugel. Anschließend erfolgt das Verschließen des Zusatzprogrammgefäßes 2 mittels eines Stopfens 16.

Nach dem Schritt des Einbringens 100 erfolgt ein Zerstören 200 des Zellmaterials 13. Dazu wird das Zusatzprobengefäß 2 alternierend bewegt, insbesondere hochfrequent alternierend bewegt. Besonders vorteilhaft ist das Zusatzprobengefäß 2 zylinderförmige ausgebildet, wobei das alternierende Bewegen des Zusatzprobengefäßes 2 parallel zu einer Mittelachse der Zylinderform erfolgt. Durch ein derartiges Bewegen des Zusatzprobengefäßes 2 wird erreicht, dass Zellen innerhalb des Zellmaterials 13 durch das Mahlelement 14 zerstört werden, wodurch die DNS aus den Zellen austreten kann.

Nach dem Durchführen des Schritts des Zerstören 200 handelt es sich bei dem Zellmaterial 13 um eine Mischung aus nicht zerstörten Zellen, zerstörten Zellen, sowie Zellinhalten, die sich innerhalb des Zusatzprobengefäßes 2 befindet. Daher erfolgt in einem weiteren Schritt ein Ausfiltern 300 des Lysats 11 in ein Probengefäß 1. Zum Ausfiltern 300 erfolgt ein Entfernen 310 des Stopfens 16. Statt dem Stopfen 16 wird ein Stempel 5 verwendet, so dass nach dem Entfernen 310 ein Aufbringen 320 des Stempels 5 erfolgt. Der Stempel 5 ist, wie bevorzugt auch das Zusatzprobengefäß 2, zylinderförmige ausgebildet. Somit ist der Stempel 5 vorteilhafterweise in das Zusatzprobengefäß 2 einführbar. Dabei entsteht eine fluiddichte Abdichtung zwischen einer Außenwand des Stempels 5 und einer Innenwand des Zusatzprobengefäßes 2.

Der Stempel 5 weist bevorzugt eine Öffnung 17 auf. Die Öffnung 17 stellt somit eine Verbindung des inneren des Zusatzprobengefäßes 2 zu einer Umgebung dar. Die Öffnung stellt insbesondere einen sich längs entlang einer Mittelachse der Zylinderform des Stempels 5 erstreckenden Kanal dar, sodass der Stempel 5 die Form eines Hohlzylinders aufweist. In der Öffnung 17 ist ein Lysatfilterelement 4 angeordnet, sodass sämtliches Fluid, dass durch den Stempel 5 in das Zusatzprobengefäß 2 gelangen soll, oder aus dem Zusatzprobengefäß 2 entfernt werden soll, durch die Öffnung 17 und das Lysatfilterelement 4 verlaufen muss.

Durch das Zusammensetzen von Zusatzprobengefäß 2 und Stempel 5 entsteht insbesondere eine Spritze. Somit ist durch relatives Bewegen von Zusatzprobengefäß 2 und Stempel 5 zueinander ermöglichte, Fluid in das Zusatzprobengefäß 2 anzusaugen oder aus dem Zusatz Druckgefäß 2 auszupressen. Dies vereinfacht das Zuführen und Abführen von Fluid in oder aus dem Zusatzprobengefäß 2.

Nach dem Aufbringen 320 des Stempels 5 ist das Zusatzprobengefäß 2 fluiddicht verschlossen, solange keine Relativbewegung zwischen Stempel 5 und Zusatzprobengefäß 2 auftritt. Somit erfolgt ein Rotieren 330 der Kombination von Zusatzprobengefäß 2 und Stempel 5, ohne dass die Gefahr besteht, dass der Inhalt des Zusatzprobengefäßes 2 herausläuft. Schließlich erfolgt ein Auspressen 340 des Satz 11 aus dem Zusatzprobengefäß 2 in das Probengefäß 1. Das Auspressen 340 wird dadurch initiiert, dass Kolben 5 und Zusatzprobengefäß 2 relativ aufeinander zubewegt werden. Aufgrund des Lysatfilterelements 4 gelangt ausschließlich das Lysat 11 aus dem Zusatzprobengefäß 2 durch den Stempel 5 in das Probengefäß 1. Das restliche Zellmaterial 13, das bedeutet insbesondere noch intakte Zellen sowie grober Zellschrott, werden ebenso wie das Mahlelement 14 durch das Lysatfilterelement 4 zurückgehalten. Somit bleiben sämtliche unerwünschten Komponenten innerhalb des Zusatzprobengefäßes 2, während lediglich das Lysat 11 in das Probengefäß 1 gelangt.

Nach dem Bereitstellen 400 des Lysats 11 erfolgt der Waschvorgang 900 des Lysats 11. Der Waschvorgang 900 umfasst wiederum eine Vielzahl von Unterschritten. Dabei ist vorgesehen, dass zu Beginn des Waschvorgangs 900 das Lysat 11 in einem frischen Probengefäß 1 bereitgestellt ist, wobei die in dem Lysat 11 enthaltene DNS das Probengefäß 1 bis zum Beenden des Waschvorgangs 900 nicht mehr verlässt.

Zunächst erfolgt ein zufügen 500 einer DNS adsorbierenden Substanz 15 in das Probengefäß 1. Bei der DNS adsorbierenden Substanz 15 handelt es sich insbesondere um Siliziumdioxid. Die DNS adsorbieren der Substanz 15 liegt insbesondere in einer Suspension vor. Auf diese Weise ist die Dosierung der DNS adsorbieren Substanz 15 vereinfacht. Anschließend erfolgt ein Verschließen 600 des Probengefäßes 1 mittels eines Stempels 5, der ein Waschfilterelement 3 umfasst. Der Stempel 5 ist insbesondere identisch aufgebaut wie der zuvor verwendete Stempel 5. Besonders vorteilhaft ist außerdem vorgesehen, dass das Waschfilterelement 3 identisch zu dem Waschfilterelement 4 ist. Somit wird für das gesamte Verfahren, das bedeutet insbesondere für das Bereitstellen 400 und für den Waschvorgang 900, lediglich ein einziger Typ von Stempeln 5 verwendet. Mit dem kombinieren von Stempel 5 und Probengefäß 1 ist das Probengefäß 1 fluiddicht verschlossen. Einzuführen und Abführen von Fluid ist lediglich durch Bewegen von Stempel 5 und Probengefäß 1 relativ zueinander ermöglicht, wobei das Fluid durch den Stempel 5 und durch das Waschfilterelement 3 geleitet wird.

Nach diesen Vorbereitungsmaßnahmen erfolgt ein, insbesondere wiederholtes, Zuführen 700 eines Waschfluides 12 und Abführen 800 des Waschfluides 12. Insbesondere erfolgt ein Zuführen 700 von unterschiedlichen Waschfluiden 12, wobei jedes Waschfluid 12 erst dann zugeführt wird, wenn das vorherige Waschfluid 12 durch den Schritt des Abführens 800 vollständig aus dem Probengefäß 1 entfernt wurde. Durch die Verwendung der unterschiedlichen Waschfluide 12 ist sichergestellt, dass sämtlicher verbleibender Zellschrott aus dem Lysat 11 entfernt wird, um eine möglichst reine DNS zu erhalten. Die DNS wird bei jedem Abführen 800 von Waschfluid 12 aufgrund des DNS adsorbierenden Materials 15 von dem Waschfilterelement 12 Rück gehalten und verbleibt somit während des gesamten Verfahrens innerhalb des Probegefäßes 1.

Um zuletzt ausschließlich DNS bereitzustellen, erfolgt eine Aufnahme einer Zusatzsubstanz in das Probengefäß 1. Die Zusatzsubstanz dient zum Lösen der DNS adsorbierenden Substanz 15 von der DNS. Somit besteht eine maximal reine DNS innerhalb des Probengefäß 1 zur Verfügung.

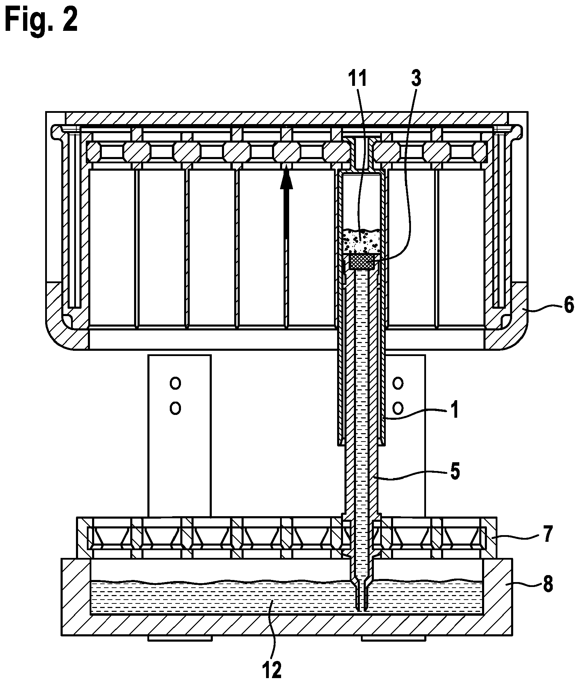

Das zuvor beschriebene Verfahren wird insbesondere automatisiert durchgeführt, wobei eine Vielzahl von Zusatzprobengefäßen 2 und/oder Probengefäßen 1 gleichzeitig verarbeitet werden. Dazu wird ein Halteelement 6 (vergleiche Figur 2) verwendet, dass eine Vielzahl von Probengefäßen 1 aufnehmen kann. Ebenso wird ein zu dem Halteelement 6 bevorzugt identisch ausgebildetes Zusatzhalteelement (nicht gezeigt) verwendet, dass eine Vielzahl von Zusatzprobengefäßen 2 aufnehmen kann. Auf diese Weise ist eine Vielzahl von Probengefäßen 1 und/oder Zusatzprobengefäßen 2 handhabbar.

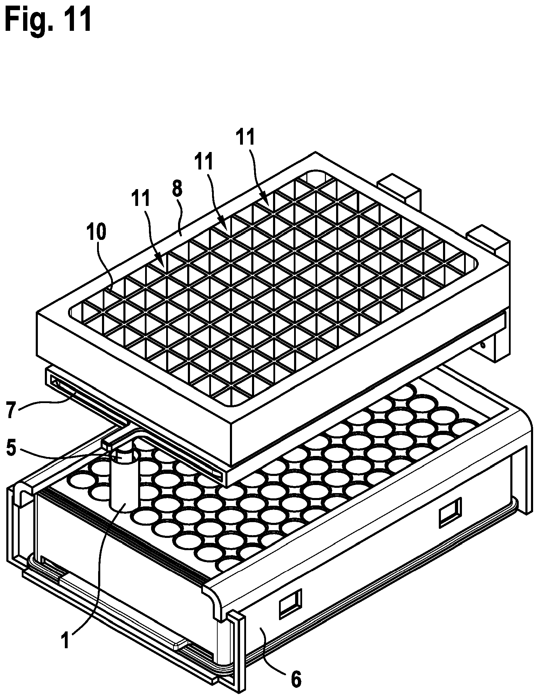

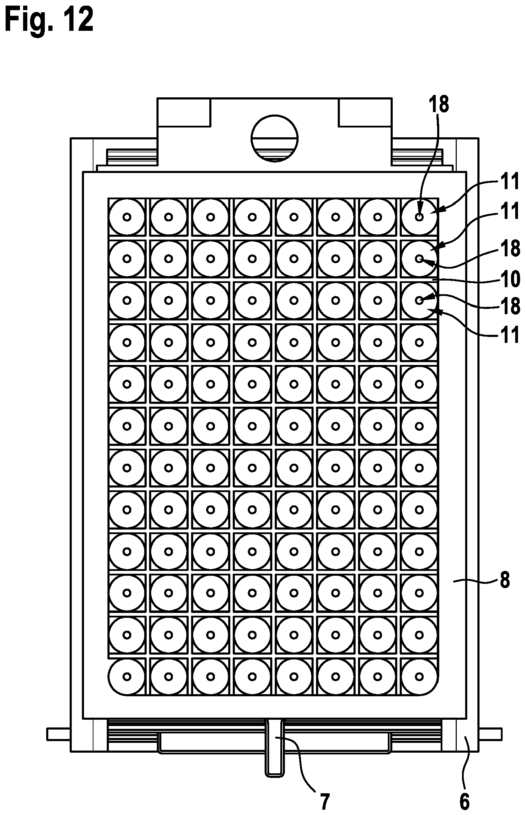

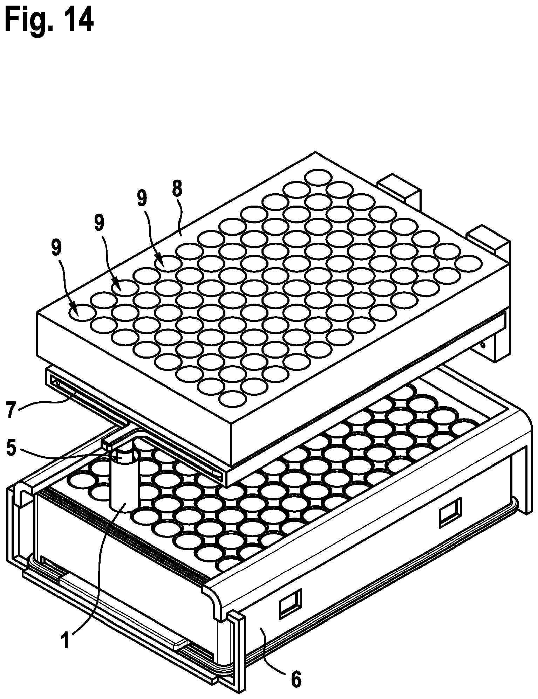

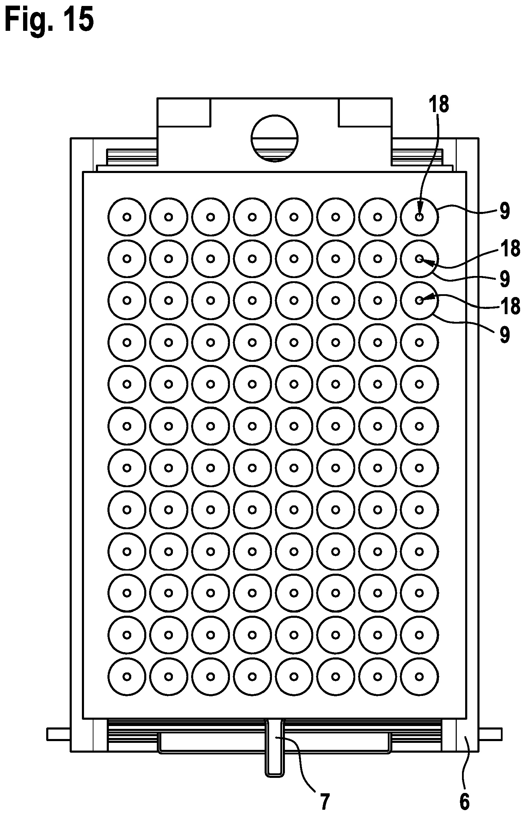

Des Weiteren wird ein Trägerelement 7 (vergleiche Figur 2) verwendet, um eine Vielzahl von Stempeln 5 zu halten. Auf diese Weise ist eine Vielzahl von Stempeln 5 eine Vielzahl von Zusatzprobengefäßen 2 gleichzeitig einsetzbar, um somit gleichzeitig aus einer Vielzahl von Zusatzprobengefäßen 2 Lysat 11 in eine Vielzahl von Probengefäßen 1 auszufiltern. Ebenso ist das Trägerelement 7 dazu verwendbar, um eine Vielzahl von Stempeln 5 gleichzeitig in die Vielzahl von Probengefäßen 1 einzusetzen. Auf diese Weise wird erreicht, dass der Waschvorgang 900 für die Vielzahl von Probengefäßen 1 gleichzeitig durchgeführt werden kann. Dies ist nachfolgend in den Figuren 2 bis 15 gezeigt.

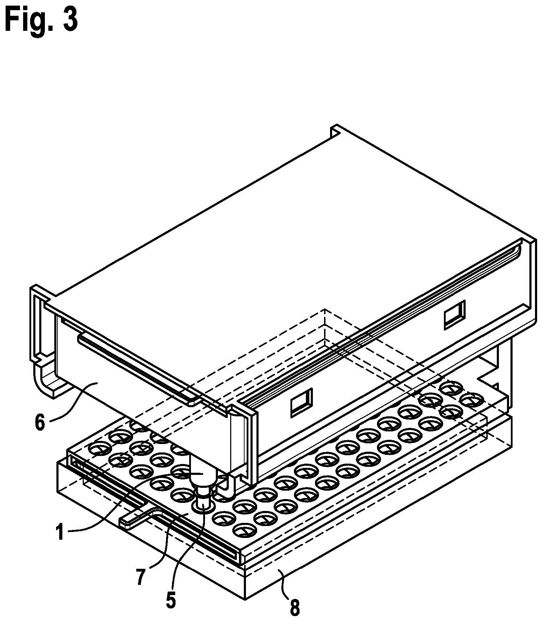

Die Figuren 2 und 3 zeigen eine erste Alternative des Waschvorgangs 900. Bei der ersten Alternative erfolgt ein anzusaugen von Waschfluid 12 aus einem gemeinsamen Reservoir 8. In das gemeinsame Reservoir 8 taucht die Vielzahl von Stempeln 5. Dazu wird insbesondere das Trägerelement 7 auf das Reservoir 8 aufgesetzt. Anschließend erfolgt eine Relativbewegung zwischen Halteelement 6 und Trägerelement 7, bei der das Halteelement 6 von dem Trägerelement 7 wegbewegt wird. Auf diese Weise wird ein Unterdruck innerhalb der Probengefäße 1 erzeugt, wodurch das Waschfluid 12 durch Stempel 5 in die Probengefäße 1 gesaugt wird. Dort vermischt sich das Waschfluid 12 mit dem Lysat 11 und wird anschließend durch eine erneute Relativbewegung zwischen Halteelement 6 und Trägerelement 7 aus dem Probengefäß 1 ausgepresst. Dazu wird das das Halteelement 6 relativ zu dem Trägerelement 7 hin bewegt. Besonders vorteilhaft ist dabei vorgesehen, dass ein Luftpolster innerhalb des Probengefäßes 1 verbleibt, so dass das Waschfluid 12 zusammen mit den auszuwaschenden Fremdsubstanzen vollständig auspressbar ist.

In der ersten Alternative ist das Reservoir 8 in einer Pfanne angeordnet, so dass die Vielzahl von Stempeln 5 von oben in das Reservoir 8 eingreifen können, um in das Waschfluid 12 einzutauchen. Stempel 5 tauchen dabei in dasselbe Waschfluid 12 ein und entnehmen somit das Waschfluid 12 aus demselben Reservoir 8.

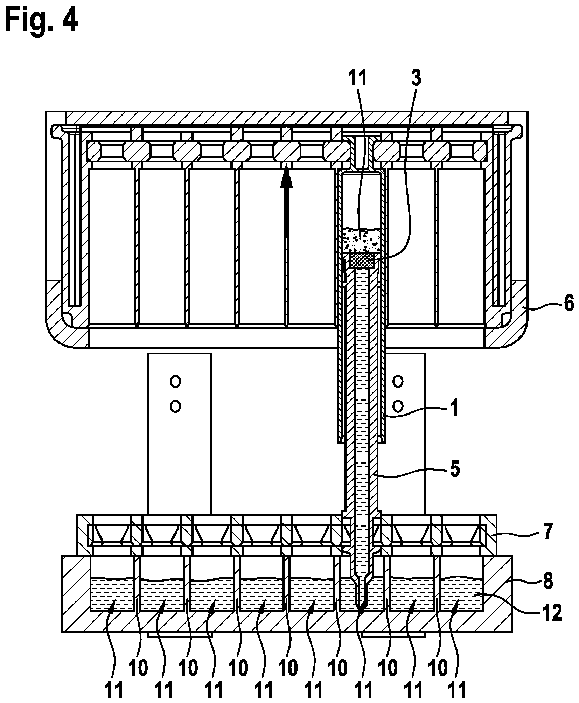

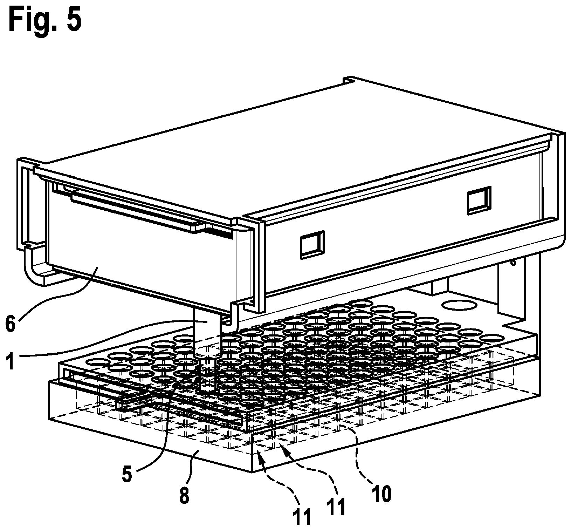

Die Figuren 4 und 5 zeigen eine zweite Alternative des Waschvorgangs 900. Dabei entspricht die zweite Alternative grundsätzlich der ersten Alternative, mit dem einzigen Unterschied, dass das Reservoir 8 mit zumindest einem, insbesondere herausnehmbar, Unterteilungselement 10 in einzelne Teilreservoirs 11 abgegrenzt ist. Somit ist insbesondere das Reservoir 8 einfach zu früh, in dem lediglich ein einziges Becken mit dem Waschfluid 12 zu versehen ist. Anschließend kann das Unterteilungselement 10 eingesetzt werden. Durch das Unterteilungselement ist das Reservoir 8 eine Vielzahl von Teilreservoirs 11 aufgeteilt, wobei Stempel 5 ein eigenes Teilreservoir 11 zur Verfügung steht. Alternativ ist ebenso möglich, dass jedes Teilreservoir 11 eigens befüllt wird, um somit eine vorgegebene Menge an Waschfluid 12 für jeden einzelnen Stempel 5 individuell einzustellen.

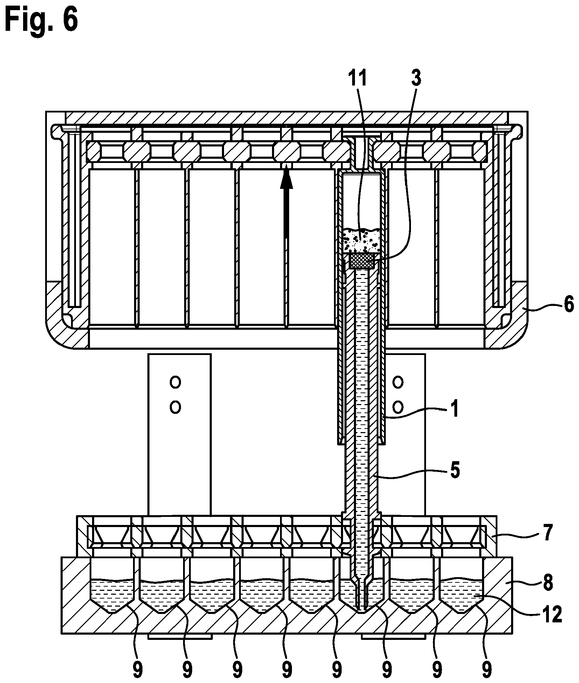

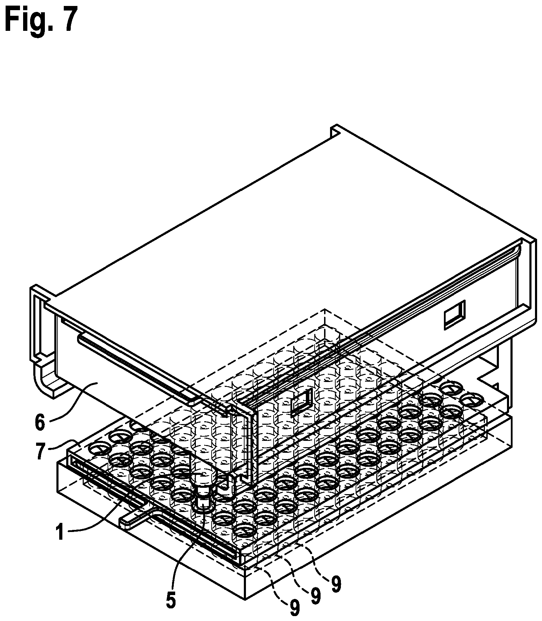

Die Figuren 6 und 7 zeigen eine dritte Alternative des Waschvorgangs 900. Im Unterschied zu der ersten Alternative ist nicht ein einzelnes Reservoir 8 vorhanden, sondern es ist eine Vielzahl von Einzelreservoirs 9 vorhanden somit taugt jeder Stempel 5 in ein eigenes Einzelreservoir 9 ein. Die dem jeweiligen Probengefäß 1 zuzuführende Menge an Waschfluid 12 ist somit jeweils einzeln einzustellen.

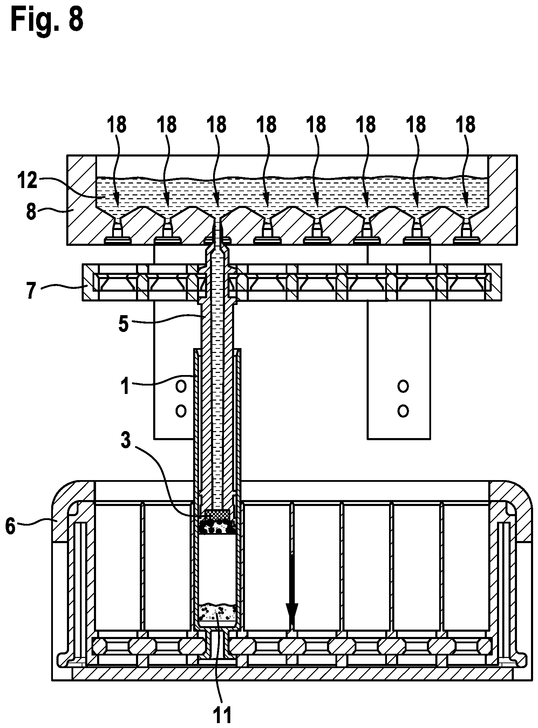

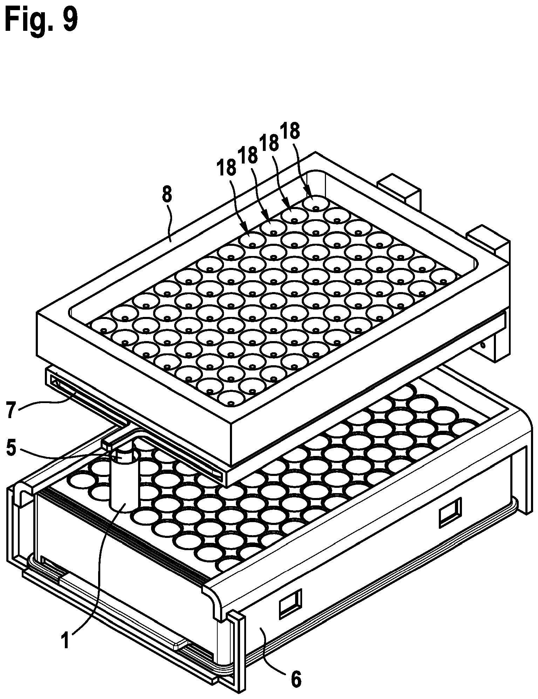

Die Figuren 8 und 9 zeigen eine vierte Alternative des Waschvorgangs 900. Wiederum ist vorgesehen, dass ein einziges Reservoir 8 vorhanden ist, dass mit dem Waschfluid 12 gefüllt ist. Aus diesem gemeinsamen Reservoir 8 wird das Waschfluid 12 für jedes einzelne Probegefäß einsenden, in dem sämtliche Stempel 5 in das gemeinsame Waschfluid 12 eintauchen. Im Gegensatz zu der vorgeschriebenen ersten Alternative erfolgt ein eintauchen jedoch nicht von oben in das Reservoir 8, sondern es ist vielmehr in einem Boden des Reservoirs 8 ein Durchgang 18 vorgesehen, durch den die Vielzahl von Stempeln 5 dringen kann. Somit ist jedem Stempel 5 ein eigener Durchgang 18 zugeordnet. Die Vielzahl von durchgängigen 18 ist insbesondere fluiddicht, solange kein Stempel 5 durch den Durchgang 18 durchdringt. Ein Befüllen des Reservoirs 8 erfolgt vorteilhafterweise von oben, sodass eine Entnahmerichtung und eine Befüllungsrichtung des Reservoirs 8 getrennt sind. Abgesehen von der unterschiedlichen Entnahmerichtung funktioniert das Entnehmen von Waschfluid 12 analog zu der ersten Alternative.

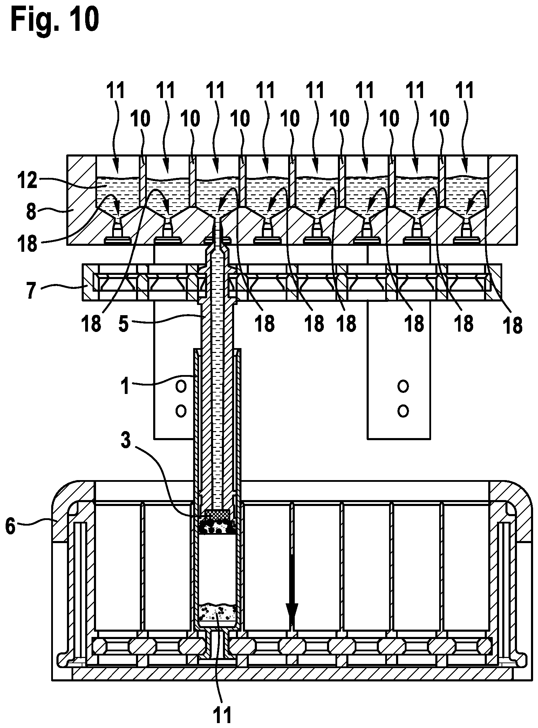

Die Figuren 10, 11 und 12 zeigen eine fünfte Alternative des Waschvorgangs 900. Hierbei ist wiederum vorgesehen, dass das gemeinsame Reservoir 8 durch zumindest ein Unterteilungselement 10 in eine Vielzahl von Teilreservoirs 11 unterteilt ist. Jedes der Reservoirs 11 weist einen Durchgang 18 zuvor beschrieben auf. Somit erfolgt ein Entnehmen von Waschfluid 12 wiederum von unten durch einen Boden des Reservoirs 8. Ansonsten unterscheidet sich der Waschvorgang 900 gemäß der fünften Alternative nicht von der zweiten Alternative.

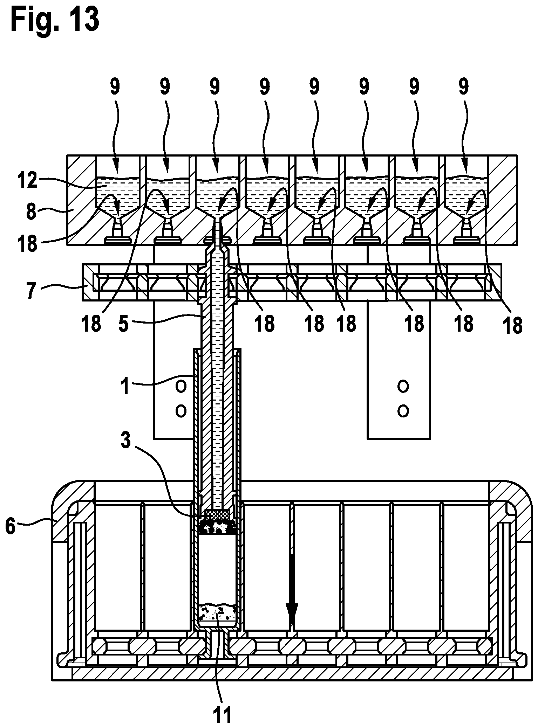

Die Figuren 13, 14 und 15 zeigen eine sechste Alternative des Waschvorgangs 900. Hierbei ist wiederum vorgesehen, dass eine Vielzahl von Einzelreservoirs 9 vorhanden ist, wobei jedes Einzelreservoir 9 einen zuvor beschriebenen Durchgang 18 aufweist. Somit kann jeder Stempel 5 durch den entsprechenden Durchgang 18 Waschfluid 12 aus dem zugehörigen Einzelreservoir 9 nehmen. Im Gegensatz zu der dritten Alternative ist somit wiederum ein Entnehmen von Waschfluid 12 durch den Boden der Vielzahl von Einzelreservoirs 9 vorgesehen. Ansonsten unterscheidet sich der Waschvorgang 900 nach der sechsten Alternative nicht von dem Waschvorgang 900 nach der dritten Alternative.

Wird das Waschfluid 12 wie in der ersten Alternative, der zweiten Alternative und der dritten Alternative von oben entnommen, so sind Vorkehrungen gegen Kontamination des Waschfluides 12 zu treffen. Gleichzeitig ist jedoch eine sehr genaue Mengenzuordnung von Waschfluid 12 zu den jeweiligen Proben gewesen 1 ermöglicht. Wird hingegen das Waschfluid wie in der vierten Alternative, der fünften Alternative und sechsten Alternative von unten entnommen, so kann das Reservoir 8 oder die Einzelreservoir 9 vollständig verschlossen und somit gegenüber der Umgebung hermetisch abgeriegelt sein. Waschfluid 12 ist erst dadurch entnehmbar, dass die Vielzahl von Stempeln durch die Durchgänge 18 dringt. Das Reservoir 8 und/oder das Unterteilungselement 10 und/oder die Einzelreservoirs 9 können bevorzugt mehrfach verwendet werden. Zwischen den einzelnen Verwendungen ist insbesondere ein Spülvorgang und/oder ein Reinigungsvorgang durchführbar.

In allen Alternativen ist bevorzugt vorgesehen, dass das Abführen 800 des Waschfluids 12 durch Auspressen des Waschfluids 12 in einen gesonderten Abfallbehälter erfolgt. Auf diese Weise ist die Kontaminationsgefahr gesenkt.

Die Vielzahl von Probengefäß 1 verbleibt vorteilhafterweise während des gesamten Waschvorgangs 900 innerhalb des Halteelements 6. Dazu sind die Probengefäß 1 an dem Halteelement 6 fixierbar. Insbesondere erfolgt ein formschlüssiges Fixieren, wobei das formschlüssige Fixieren lösbar ist. Auf diese Weise lässt sich die Vielzahl von Probengefäßen 1, die von dem Halteelement 6 gehalten werden, einfach und aufwandsarm handhaben. Insbesondere ist ein gleichzeitiges Durchführen eines Waschvorgangs 900 für eine Vielzahl von Probengefäßen 1 durchführbar. Somit wird insbesondere erreicht, dass ein Durchsatz bei dem Extrahieren von DNS maximiert ist. Besonders vorteilhaft kann jedes Halteelement 6 eine Anzahl von 96 Probengefäßen 1 aufnehmen.

Ebenso wie für den Waschvorgang 900 ist bevorzugt vorgesehen, dass auch der Schritt des Bereitstellens 400 des Lysats 11 für eine Vielzahl von Zusatzprobengefäßen 2 gleichzeitig durchgeführt wird. Die Zusatzprobengefäße 2 werden einem dem Halteelement 6 baugleichen Zusatzhalteelement (nicht gezeigt) gehalten, wobei auch das Zusatzhalteelement bevorzugt 96 Zusatzprobengefäße 2 aufnehmen kann. Auf diese Weise ist insbesondere der Vorgang des Zerstörens 200 des Zellmaterials 13 vereinfacht, da eine Vielzahl von Zusatzprobengefäßen 2 gleichzeitig bewegt werden kann, indem das Zusatzhalteelement bewegt wird.

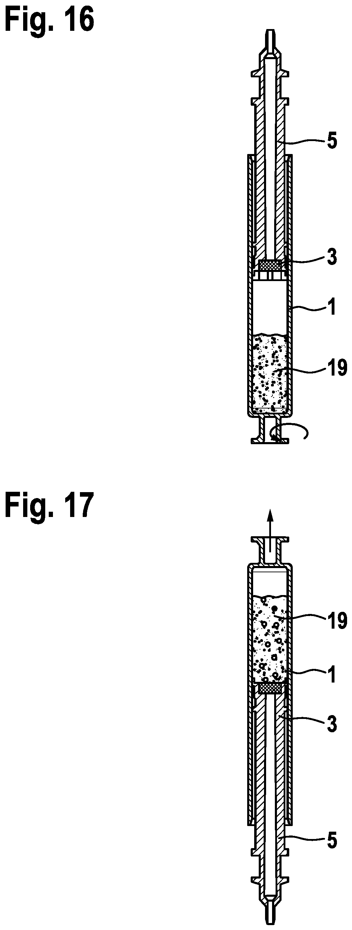

Um ein effizientes Mischen des Waschfluids 12 und des Lysats 11 zu einem Reinigungsgemisch 19 zu ermöglichen, ist vorteilhafterweise vorgesehen, dass ein Bewegen des Probengefäßes 1 erfolgt. Dies ist in Figur 16 dargestellt. Das Probengefäß 1 wird insbesondere hochfrequent kreisförmig bewegt. Auf diese Weise findet optimales Durchmischen statt. Ebenso werden auf diese Weise an dem Waschfilterelement 3 anhaftende Partikel gelöst und mit dem Reinigungsgemisch 19 vermischt. Bei solchen anhaftenden Partikeln handelt es sich insbesondere um DNS, die aufgrund der DNS adsorbierenden Substanz 15 von dem Waschfilterelement 3 zurückgehalten wurde. Durch das Vermischen mit dem Reinigungsgemisch 19 ist somit sichergestellt, dass die DNS hochwertig gereinigt wird.

In Figur 17 ist eine weitere Möglichkeit gezeigt, Partikel von dem Waschfilterelement 3 zu lösen. Dazu wird nach dem Zuführen 800 des Waschfluids 12 durch Ansaugen durch den Stempel 5 auf dieselbe Art und Weise Luft durch den Stempel 5 angesaugt. Dies erfolgt somit durch eine Relativbewegung zwischen Stempel 5 und Probengefäß 1, besonders vorteilhaft durch die Relativbewegung zwischen Halteelement 6 und Trägerelement 7. Durch die Auflockerung der Partikel von dem Waschfilterelement 3 kann die Effizienz des Waschvorgangs 900 erhöht werden.

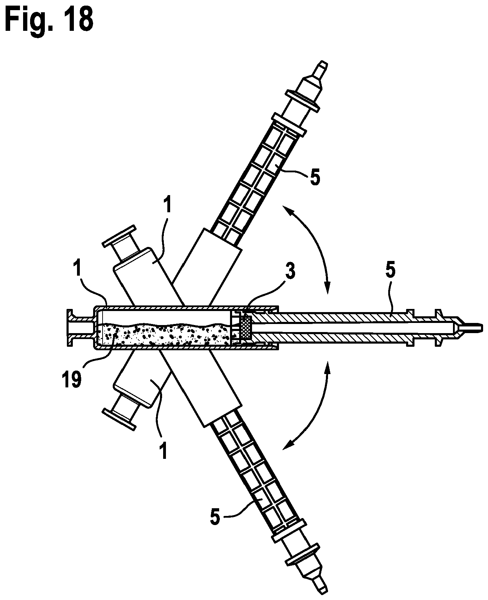

Schließlich zeigt Figur 18 eine weitere Möglichkeit, Partikel von dem Waschfilterelement 3 zu lösen und eine optimale Durchmischung des Reinigungsgemisches 19 sicherzustellen. Dazu erfolgt ein alternierend des Verschwenken des Probengefäßes 1 sowie des Stempels 5. Da das Probengefäß 1 durch den Stempel 5 fluiddicht verschlossen ist, ist hierbei ein Austreten von Fluid ausgeschlossen.

Besonders vorteilhaft ist während des gesamten Waschvorgangs 900 die DNS ausschließlich innerhalb des Probengefäßes 1 vorhanden. Eine Handhabung der DNS ist somit sehr einfach möglich und damit optimal automatisierbar. Durch das einfache durchführen des Waschvorgangs 900 durch wiederholtes relatives Bewegen von Halteelement 6 und Trägerelement 7 ist auch der Waschvorgang 900 sehr einfach und aufwandsarm automatisierbar. Dasselbe gilt für Vorrang des Bereitstellens 400 des Lysats 4. Aufgrund der einfachen Automatisierbarkeit lassen sich somit eine große Vielzahl von Extraktionen von DNS gleichzeitig durchführen. Dabei läuft das gesamte Verfahren in sequenziellen Schritten ab und ist damit einfach und aufwandsarm zu kontrollieren und zu überwachen. Gleichzeitig ist aufgrund des parallelen Durchführens der Schritte ein hoher Durchsatz gewährleistet. Schließlich ist vorteilhafterweise vorgesehen, dass aufgrund des beschriebenen Verfahrens gemäß dem Ausführungsbeispiel der Erfindung hochreine DNS extrahierbar ist, die für weitere Schritte zur Verfügung gestellt werden kann.

## Patentansprüche

1. Verfahren zum Extrahieren von DNS, umfassend die Schritte:
• Bereitstellen (400) eines Lysats (11) in einem Probengefäß (1),
• Zufügen (500) einer DNS adsorbierenden Substanz (15) zu dem Probengefäß (1),
• Verschließen (700) des Probengefäßes (1) mittels eines Waschfilterelements (3),
• Zuführen (600) eines Waschfluids (12) in das Probengefäß (1) durch das Waschfilterelement (3), und
• Abführen (700) des Waschfluids (12) aus dem Probengefäß (1) durch das Waschfilterelement (3),
• wobei die DNS adsorbierende Substanz von dem Waschfilterelement (3) rückhaltbar ist.

2. Verfahren nach Anspruch 1, **dadurch gekennzeichnet, dass** der Schritt des Bereitstellens des Lysats (11) die folgenden Schritte umfasst:
• Einbringen (100) von Zellmaterial (13) sowie eines Mahlelements (14), insbesondere einer Mahlkugel, in ein Zusatzprobengefäß (2),
• Zerstören (200) des Zellmaterial (13) durch Bewegen des Zusatzprobengefäßes (2), insbesondere durch hochfrequentes alternierendes Bewegen des Zusatzprobengefäßes (2), und
• Ausfiltern (300 des Lysats (11) aus dem Zusatzprobengefäß (2) in das Probengefäß (1) durch ein Lysatfilterelement (4).

3. Verfahren nach einem der vorhergehenden Ansprüche, **dadurch gekennzeichnet, dass** das Waschfilterelement (3) oder das Lysatfilterelement (4) in einem Stempel (5) angeordnet ist, wobei der Stempel (5) in das Probengefäß (1) oder das Zusatzprobengefäß (2) einsteckbar ist und wobei durch den Stempel (5)
• das Waschfluid (12) in das Probengefäß (1) gesaugt und aus dem Probengefäß (1) gepresst wird, oder
• das Lysat (11) von dem Zusatzprobengefäß (2) in das Probengefäß (1) gepresst wird.

4. Verfahren nach Anspruch 3, **dadurch gekennzeichnet, dass** der Stempel (5) und das Probengefäß (1) oder das Zusatzprobengefäß (2) eine Spritze bilden.

5. Verfahren nach einem der vorhergehenden Ansprüche, **dadurch gekennzeichnet, dass** das Probengefäß (1) während des gesamten Verfahrens in einer Haltevorrichtung (6) gehalten wird und/oder das Zusatzprobengefäß (2) zumindest während des Schritts des Bereitstellens (400) des Lysats (11) in einer Zusatzhaltevorrichtung gehalten wird.

6. Verfahren nach Anspruch 5, **dadurch gekennzeichnet, dass** das Halteelement (6) eine Vielzahl von Probengefäßen (1) gleichzeitig hält und/oder das Zusatzhaltelement eine Vielzahl von Zusatzprobengefäßen (2) gleichzeitig hält, wodurch das Verfahren auf der Vielzahl von Probengefäßen (1) und/oder Zusatzprobengefäßen (2) gleichzeitig angewandt wird.

7. Verfahren nach Ansprüchen 4 und 5 oder 4 und 6, **dadurch gekennzeichnet, dass** eine Vielzahl von Stempeln (5) an einem Trägerelement (7) fixiert sind, sodass gleichzeitig die Vielzahl von Stempeln (5) gleichzeitig in die Vielzahl von Probengefäßen (1) und/oder Zusatzprobengefäßen (2) eingesteckt werden.

8. Verfahren nach Anspruch 7, **dadurch gekennzeichnet, dass** das Zuführen (800) von Waschfluid (12) und/oder das Abführen (900) von Waschfluid (12) und/oder das Ausfiltern (400) des Lysats (11) durch Bewegen des Trägerelements (7) und des Halteelements (6) und/oder des Zusatzhalteelements relativ zueinander erfolgt.

9. Verfahren nach Anspruch 7 oder 8, **dadurch gekennzeichnet, dass** das Zuführen (800) von Waschfluid (12) durch gleichzeitiges Ansaugen von Waschfluid (12) über die Vielzahl von Stempeln (5) aus einem gemeinsamen Reservoir (8) oder aus einem für jeden Stempel (5) vorgesehenen Einzelreservoir (9) erfolgt.

10. Verfahren nach Anspruch 9, **dadurch gekennzeichnet, dass** das Reservoir (8) durch zumindest ein, insbesondere herausnehmbares, Unterteilungselement (10) in einzelne Teilreservoirs (11) für jeden Stempel (5) unterteilt wird.

11. Verfahren nach einem der vorhergehenden Ansprüche, **dadurch gekennzeichnet, dass** die Schritte des Zuführens (800) von Waschfluid (12) und des Abführens (900) von Waschfluid (12) wiederholt durchgeführt werden.

12. Verfahren nach einem der vorhergehenden Ansprüche, **dadurch gekennzeichnet, dass** nach dem Zuführen (800) des Waschfluids (12) und vor dem Abführen (900) des Waschfluids (12) dem Probengefäß (1) Luft zugeführt wird, wobei die Luft dem Probengefäß (1) insbesondere auf dieselbe Weise wie das Waschfluids (12) zugeführt wird.

13. Verfahren nach einem der vorhergehenden Ansprüche, **dadurch gekennzeichnet, dass** nach dem Zuführen (800) des Waschfluids (12) und vor dem Abführen (900) des Waschfluids (12) das Probengefäß (1), insbesondere hochfrequent, kreisförmig oder alternierend bewegt wird.

14. Verfahren nach einem der vorhergehenden Ansprüche, **dadurch gekennzeichnet, dass** das Waschfilterelement (3) und das Lysatfilterelement (4) identisch sind und/oder das Probengefäß (1) und das Zusatzprobengefäß (2) identisch aufgebaut sind und/oder das Halteelement (6) und das Zusatzhalteelement identisch aufgebaut sind.

15. Verfahren nach einem der vorhergehenden Ansprüche, **dadurch gekennzeichnet, dass** nach dem Abführen (900) des Waschfluids (12) eine Zusatzsubstanz in das Probengefäß (1) eingegeben wird, um die DNS adsorbierende Substanz (15) von der DNS zu lösen.

## Claims

1. A method for extracting DNA, comprising the steps of:
• providing (400) a lysate (11) in a sample vessel (1),
• adding (500) a DNA-adsorbing substance (15) to the sample vessel (1),
• closing (700) the sample vessel (1) by means of a wash filter element (3),
• feeding (600) a wash fluid (12) into the sample vessel (1) through the wash filter element (3), and
• discharging (700) the wash fluid (12) from the sample vessel (1) through the wash filter element (3),
• wherein the DNA-adsorbing substance can be retained by the wash filter element (3).

2. The method according to claim 1, **characterized in that** the step of providing the lysate (11) comprises the steps of:
• introducing (100) cellular material (13) and a grinding element (14), in particular a grinding ball, into an additional sample vessel (2),
• breaking down (200) the cellular material (13) by moving the additional sample vessel (2), in particular by high-frequency alternating movement of the additional sample vessel (2), and
• filtering out (300) the lysate (11) from the additional sample vessel (2) into the sample vessel (1) through a lysate filter element (4).

3. The method according to any one of the preceding claims,
**characterized in that** the wash filter element (3) or the lysate filter element (4) is arranged in a plunger (5), wherein the plunger (5) is insertable into the sample vessel (1) or the additional sample vessel (2) and wherein, by means of the plunger (5),
• the wash fluid (12) is sucked into the sample vessel (1) and pressed out of the sample vessel (1), or
• the lysate (11) is pressed out from the additional sample vessel (2) into the sample vessel (1).

4. The method according to claim 3, **characterized in that** the plunger (5) and the sample vessel (1) or the additional sample vessel (2) form a syringe.

5. The method according to any one of the preceding claims,
**characterized in that** the sample vessel (1) is held in a holding device (6) throughout the entire method, and/or the additional sample vessel (2) is held in an additional holding device at least during the step of providing (400) the lysate (11).

6. The method according to claim 5, **characterized in that** the holding element (6) simultaneously holds a plurality of sample vessels (1) and/or the additional holding element simultaneously holds a plurality of additional sample vessels (2), whereby the method is applied simultaneously to the plurality of sample vessels (1) and/or additional sample vessels (2).

7. The method according to claims 4 and 5 or 4 and 6, **characterized in that** a plurality of plungers (5) are fixed on a carrier element (7), so that, at the same time, the plurality of plungers (5) are simultaneously inserted into the plurality of sample vessels (1) and/or additional sample vessels (2).

8. The method according to claim 7, **characterized in that** the feeding (800) of wash fluid (12) and/or the discharging (900) of wash fluid (12) and/or the filtering out (400) of the lysate (11) are carried out by moving the carrier element (7) and the holding element (6) and/or the additional holding element relative to one another.

9. The method according to claim 7 or 8, **characterized in that** the feeding (800) of wash fluid (12) is carried out by simultaneously sucking wash fluid (12) through the plurality of plungers (5) from a common reservoir (8) or from an individual reservoir (9) provided for each plunger (5).

10. The method according to claim 9, **characterized in that** the reservoir (8) is subdivided by at least one, in particular removable, subdivision element (10) into individual sub-reservoirs (11) for each plunger (5).

11. The method according to any one of the preceding claims,
**characterized in that** the steps of feeding (800) wash fluid (12) and discharging (900) wash fluid (12) are carried out repeatedly.

12. The method according to any one of the preceding claims,
**characterized in that** after the wash fluid (12) is fed (800) and before the wash fluid (12) is discharged (900), air is fed to the sample vessel (1), wherein the air is fed to the sample vessel (1) in particular in the same way as the wash fluid (12).

13. The method according to any one of the preceding claims,
**characterized in that** after the wash fluid (12) is fed (800) and before the wash fluid (12) is discharged (900), the sample vessel (1) is moved circularly or alternatingly, in particular at high frequency.

14. The method according to any one of the preceding claims,
**characterized in that** the wash filter element (3) and the lysate filter element (4) are identical and/or the sample vessel (1) and the additional sample vessel (2) are constructed identically and/or the holding element (6) and the additional holding element are constructed identically.

15. The method according to any of the preceding claims, **characterized in that** after the wash fluid (12) is discharged (900), an additional substance is introduced into the sample vessel (1) in order to separate the DNA-adsorbing substance (15) from the DNA.

## Revendications

1. Procédé d'extraction d'ADN, comprenant les étapes :
- de fourniture (400) d'un lysat (11) dans un récipient pour échantillon (1),
- d'ajout (500) d'une substance absorbant l'ADN (15) au récipient pour échantillon (1),
- de fermeture (700) du récipient pour échantillon (1) au moyen d'un élément filtre de lavage (3),
- d'amenée (600) d'un fluide de lavage (12) dans le récipient pour échantillon (1) par l'intermédiaire de l'élément filtre de lavage (3), et
- d'évacuation (700) du fluide de lavage (12) du récipient pour échantillon (1) par l'intermédiaire de l'élément filtre de lavage (3),
- dans lequel la substance absorbant l'ADN peut être retenue par l'élément filtre de lavage (3).

2. Procédé selon la revendication 1, **caractérisé en ce que** l'étape de fourniture du lysat (11) comprend les étapes suivantes :
- l'introduction (100) de matériau cellulaire (13) ainsi que d'un élément de broyage (14), en particulier d'un boulet de broyage, dans un récipient pour échantillon supplémentaire (2),
- la destruction (200) du matériau cellulaire (13) par déplacement du récipient pour échantillon supplémentaire (2), en particulier par déplacement alterné à haute fréquence du récipient pour échantillon supplémentaire (2), et
- la filtration (300) du lysat (11) à partir du récipient pour échantillon supplémentaire (2) dans le récipient pour échantillon (1) par l'intermédiaire d'un élément filtre de lysat (4).

3. Procédé selon l'une quelconque des revendications précédentes, **caractérisé en ce que** l'élément filtre de lavage (3) ou l'élément filtre de lysat (4) est disposé dans un poinçon (5), dans lequel le poinçon (5) peut être introduit dans le récipient pour échantillon (1) ou le récipient pour échantillon supplémentaire (2) et dans lequel par l'intermédiaire du poinçon (5)
- le fluide de lavage (12) est aspiré dans le récipient pour échantillon (1) et pressé hors du récipient pour échantillon (1), ou
- le lysat (11) est pressé à partir du récipient pour échantillon supplémentaire (2) dans le récipient pour échantillon (1).

4. Procédé selon la revendication 3, **caractérisé en ce que** le poinçon (5) et le récipient pour échantillon (1) ou le récipient pour échantillon supplémentaire (2) forment une seringue.

5. Procédé selon l'une quelconque des revendications précédentes, **caractérisé en ce que** le récipient pour échantillon (1) est maintenu pendant tout le procédé dans un dispositif de maintien (6) et/ou le récipient pour échantillon supplémentaire (2) est maintenu au moins pendant l'étape de fourniture (400) du lysat (11) dans un dispositif de maintien supplémentaire.

6. Procédé selon la revendication 5, **caractérisé en ce que** l'élément de maintien (6) maintient une pluralité de récipients pour échantillon (1) et/ou l'élément de maintien supplémentaire maintient une pluralité de récipients pour échantillon supplémentaires (2) en même temps, ce qui a pour effet que le procédé est appliqué simultanément sur la pluralité de récipients pour échantillon (1) et/ou récipients pour échantillon supplémentaires (2).

7. Procédé selon les revendications 4 et 5 ou 4 et 6, **caractérisé en ce qu'**une pluralité de poinçons (5) sont fixés sur un élément de support (7), de sorte que simultanément la pluralité de poinçons (5) sont introduits simultanément dans la pluralité de récipients pour échantillon (1) et/ou récipients pour échantillon supplémentaires (2).

8. Procédé selon la revendication 7, **caractérisé en ce que** l'amenée (800) de fluide de lavage (12) et/ou l'évacuation (900) de fluide de lavage (12) et/ou la filtration (400) du lysat (11) s'effectue par déplacement de l'élément de support (7) et de l'élément de maintien (6) et/ou de l'élément de maintien supplémentaire les uns par rapport aux autres.

9. Procédé selon la revendication 7 ou 8, **caractérisé en ce que** l'amenée (800) de fluide de lavage (12) s'effectue par aspiration simultanée de fluide de lavage (12) par l'intermédiaire de la pluralité de poinçons (5) hors d'un réservoir (8) commun ou hors d'un réservoir individuel (9) prévu pour chaque poinçon (5).

10. Procédé selon la revendication 9, **caractérisé en ce que** le réservoir (8) est divisé par au moins un élément de division (10), en particulier amovible, en réservoirs partiels (11) individuels pour chaque poinçon (5).

11. Procédé selon l'une quelconque des revendications précédentes, **caractérisé en ce que** les étapes d'amenée (800) de fluide de lavage (12) et d'évacuation (900) de fluide de lavage (12) sont mises en œuvre à plusieurs reprises.

12. Procédé selon l'une quelconque des revendications précédentes, **caractérisé en ce qu'**après l'amenée (800) du fluide de lavage (12) et avant l'évacuation (900) du fluide de lavage (12) de l'air est amené au récipient pour échantillon (1), dans lequel l'air est amené au récipient pour échantillon (1) en particulier de la même manière que le fluide de lavage (12) .

13. Procédé selon l'une quelconque des revendications précédentes, **caractérisé en ce qu'**après l'amenée (800) du fluide de lavage (12) et avant l'évacuation (900) du fluide de lavage (12) le récipient pour échantillon (1) est déplacé de manière circulaire ou alternée, en particulier à haute fréquence.

14. Procédé selon l'une quelconque des revendications précédentes, **caractérisé en ce que** l'élément filtre de lavage (3) et l'élément filtre de lysat (4) sont identiques et/ou le récipient pour échantillon (1) et le récipient pour échantillon supplémentaire (2) sont conçus de manière identique et/ou l'élément de maintien (6) et l'élément de maintien supplémentaire sont conçus de manière identique.

15. Procédé selon l'une quelconque des revendications précédentes, **caractérisé en ce qu'**après l'évacuation (900) du fluide de lavage (12) une substance supplémentaire est entrée dans le récipient pour échantillon (1), afin de détacher la substance absorbant l'ADN (15) de l'ADN.
